# EUROPÄISCHE PATENTSCHRIFT

(11) **EP 3 132 766 B1**
(45) Veröffentlichungstag und Bekanntmachung des Hinweises auf die Patenterteilung: **07.10.2020**
(21) Anmeldenummer: 15182057.8
(22) Anmeldetag: 21.08.2015
(51) Int. Cl.: A61B 90/00, A61B 90/40, A61B 46/23, A61F 13/00, A61B 17/3203

(54) **SCHUTZABDECKUNG**
PROTECTIVE COVERING
REVETEMENT DE PROTECTION

(43) Veröffentlichungstag der Anmeldung: 22.02.2017
(73) Patentinhaber: MEDAXIS AG, 6340 Baar (CH)
(72) Erfinder: Moser, Beat, 8926 Uerzlikon (CH); Zweifel, Adrian, 8645 Jona (CH); Selek, Oezkan, 6430 Baar (CH); Widmer, Beat, 6005 Luzern (CH); Good, Roman, 8057 Zürich (CH)
(74) Vertreter: Grünecker Patent- und Rechtsanwälte PartG mbB

(56) Entgegenhaltungen:
- US-A- 4 998 538
- US-A1- 2005 222 544
- US-A1- 2009 126 741
- US-A1- 2009 326 489
- US-A1- 2011 290 257
- US-B1- 6 695 773

## Beschreibung

Die vorliegende Erfindung betrifft eine Schutzabdeckung zum Schutz vor Kontamination mit Körper- und/oder Spülflüssigkeit bei der Behandlung von Wunden oder Geschwüren. Sie betrifft ferner eine mit einer solchen Schutzabdeckung ausgerüstete Vorrichtung zum Spülen von Wunden oder Geschwüren.

Spülen ist ein häufig angewandtes Verfahren zum Reinigen offener, kontaminierter und chronischer Wunden. Beim Spülen wirken sterile Spülflüssigkeiten auf Wunden ein, um lebloses Gewebe, bakterielles Inoculum, Blutklumpen, lose Verschmutzung und fremde Körper in der Nähe und in den Tiefen der Wunde zu entfernen. Die kritischen Parameter eines jeden Wundspülverfahrens sind die Anwendung eines angepassten Volumens steriler Spülflüssigkeit auf die Wunde und die Verwendung ausreichenden Drucks, der in einem präzisen Verteilungsmuster aufgebracht werden muss, um effektiv die Kontaminanten zu entfernen.

In diesem Zusammenhang ist aus der EP 2 251 142 A1 eine Einwegdüse zum Einsetzen in ein Handstück bekannt, wobei diese Düse einen stark gebündelten Hochdruck-Mikrowasser-strahl zum Behandeln von Wunden erzeugt. Ein solcher Hochdruck-Mikrowasserstrahl kann insbesondere für das Reinigen, Auswaschen und Debridieren nekrotischer, nasser oder aus anderen Gründen schlecht heilender Wunden (z. B. herrührend von Erkrankungen wie Geschwüren, Gangränen, Dekubiti, Abszessen oder Fisteln) eingesetzt werden. Die kinetische Aufprallkraft der unter Druck stehenden Flüssigkeit wird dabei ausgenutzt, um die Wunde zu reinigen und zu debridieren. Insbesondere das Debridement, also die Sanierung des Wundbettes durch Entfernung nekrotischer und fibrinöser Beläge, erfordert den Einsatz eines mit hohem Druck betriebenen Spülflüssigkeitsstrahls.

Dokumente US2009126741 A1, US6695773 B1, US4998538 A, US2009326489 A1, US2011290257 A1, US2005222544 A1 und WO2005102185 A1 offenbaren weitere einschlägige Vorrichtungen aus dem Stand der Technik.

Der hohe Druck des Flüssigkeitsstrahls führt jedoch nicht nur zur Entfernung des Belags oder der Partikel aus der Wunde, sondern unweigerlich rund um die Wunde auch zur Bildung eines Aerosols (Flüssigkeitsnebels) aus in der Luft schwebenden Körperflüssigkeits- und Spülflüssigkeitstropfen. Aerosole können jedoch auch auf andere Art entstehen, z. B. bei einer Wundbehandlung mittels Plasma oder Ultraschall. Solche Aerosole können für den Fall, dass keine Schutzmaßnahmen getroffen werden, frei in die Umgebung entweichen und dadurch ein erhebliches Gefährdungspotential für den Patienten und/oder die jeweilige Gesundheitsversorgungsfachkraft darstellen.

Das hohe Gefährdungspotential solcher Aerosole rührt daher, dass Wunden fast immer von Krankheitserregern (wie Bakterien, Viren oder Pilzen) besiedelt sind, wodurch auch die bei ihrer Behandlung entstehenden Aerosole ein potentiell infektiöses, weil mit Erregern belastetes Medium darstellen. Infolgedessen kann es bei Fehlen zusätzlicher Schutzmaßnahmen zu gefährlichen Kreuzinfektionen zwischen dem Patienten und den im Behandlungszimmer anwesenden Personen oder den später in das Behandlungszimmer tretenden Personen oder den später die Behandlungsgeräte benutzenden Personen kommen. Zudem würde eine freie Ausbreitung des Aerosols während der Wundbehandlung zu Verschmutzungen im Behandlungszimmer, insbesondere auf dem Behandlungstisch und den umgebenden Bodenflächen, führen. Daraus resultiert wiederum eine erhöhte Rutschgefahr und somit Verletzungsrisiko für die Personen im Behandlungszimmer. Ein weiteres Problem stellt die Einschränkung der Sicht auf das Behandlungsfeld dar, wenn sich das frei ausbreitende Aerosol z. B. auf der Schutzbrille der behandelnden Person sammelt.

In Anbetracht der vorgenannten Nachteile sind aus dem Stand der Technik zahlreiche Verfahren und Vorrichtungen bekannt, um die Kontaminierung der Umgebung durch bei der Wundspülung entstehende Aerosole einzudämmen oder sogar ganz zu verhindern.

Die gängigste und einfachste Möglichkeit besteht darin, den zu behandelnden Körperteil des Patienten unter eine transparente Einwegschutzfolie zu platzieren, die unter Zuhilfenahme einer entsprechenden Ständervorrichtung aufgehängt und zeltförmig aufgespannt werden kann. Die jeweilige Gesundheitsversorgungsfachkraft zieht Schutzmantel, Schutzhandschuhe, Mundschutz und Schutzbrille an, bevor sie zur Durchführung der Wundbehandlung entweder unter die Schutzfolie oder in entsprechende, in der Schutzfolie vorgesehene Handöffnungen eingreift. Die während der Behandlung abfließende Behandlungsflüssigkeit kann mittels Kompressen und/oder einer gut absorbierenden Wundunterlage aufgefangen werden.

Solche Schutzfolien bieten jedoch nur einen unzureichenden Schutz der Gesundheitsversorgungsfachkräfte und Patienten vor dem Rückspritzen von mit Bakterien, Viren, Pilzen, Parasiten und/oder sonstigen pathogenen Krankheitserregern belasteten Körper- und Spülflüssigkeitstropfen. Zum einen können solche Erreger natürlich durch die beiden Handöffnungen in die Umgebung geraten. Zum anderen ist von Nachteil, dass die behandschuhte Hand nach wie vor in die unter der Schutzfolie gelegene, vom Aerosol kontaminierte Behandlungskammer eingreifen muss. Die Erreger können somit nach Beendigung der Behandlung über Handschuhe und Schutzkleidung in die Umgebung, bspw. an Regelknöpfe von Behandlungsgeräten, gelangen. Ebenfalls von Nachteil ist, dass sich die Sichtverhältnisse und die Spritzschutzwirkung umgekehrt proportional zueinander verhalten. Je hermetischer die Wunde von der Schutzfolie abgedeckt wird, desto eingeschränkter ist die Sicht der behandelnden Person auf die Wundstelle und umgekehrt.

Zwar sind auch Schutzfolien mit als Handschuhen ausgestalteten Fortsätzen zum Eingreifen einer behandelnden Person bekannt. Nachteilig an solchen integrierten Lösungen ist, dass sich das Zwängen der Hände in bzw. aus den Handschuhfortsätzen als sehr schwierig erweist. Zudem bleibt der Nachteil erhalten, dass die behandelnde Person in die aerosolbelastete Behandlungskammer eingreifen muss. Auch können pathogene Krankheitserreger (wie z. B. Bakterien) durch die Handschuhfortsätze weiterhin in die Umgebung gelangen.

Ferner sind aus dem Stand der Technik Saug-/Spülspitzen bekannt, die am vorderen (distalen) Ende über eine normalerweise trichterförmig ausgebildete Spritzblende verfügen, um ein Rückspritzen der Spülflüssigkeit zu verhindern (siehe z. B. US 5 460 604 A). Nachteilig an solchen Vorrichtungen ist, dass die während der Wundbehandlung von der Saug-/Spülspitze auszuführende Bewegung auch von der bündig auf der Haut des Patienten aufliegenden Spritzblende vollzogen werden muss. Diese Bewegung kann vom Patienten als schmerzhaft empfunden werden. Wird die Spritzblende aber von der Haut abgehoben, um dieses Schmerzempfinden zu beseitigen, so wird auch die Dichtigkeit der unter der Spritzblende gebildeten Behandlungskammer herabgesetzt. Gefährliche, hochkontagiöse Flüssigkeitstropfen können somit trotz Spritzblende in die Umgebung entweichen.

Darüber hinaus sind Anlagen bekannt, bei denen eine Flüssigkeitsstrahleinrichtung mit einer Absaugeinrichtung zur Absaugung der abgetrennten oder gelösten Gewebezellen und/oder der Spülflüssigkeit kombiniert ist. Derartige Anlagen sind aber sehr aufwändig aufgebaut, da nicht nur eine Flüssigkeitszufuhr, sondern auch eine zusätzliche Absaugung (ggf. mit einer Rückführung der Spülflüssigkeit) vorhanden sein muss.

Die Erfindung ist in dem angefügten unabhängigen Anspruch 1 offenbart. Vorteilhafte Ausführungsformen sind in den abhängigen Ansprüchen offenbart.

J Aufgabe der vorliegenden Erfindung ist es daher, eine Schutzabdeckung bereitzustellen, die auf einfache Weise einen sicheren Schutz gegen Kontamination bietet, ohne dass sie die Effektivität der Wundbehandlung beeinträchtigt.

Gelöst wird diese Aufgabe durch eine Schutzabdeckung mit den Merkmalen des Anspruchs 1.

Die erfindungsgemäße Schutzabdeckung zum Schutz vor Kontamination mit Körper- und/ oder Spülflüssigkeit bei der Behandlung von Wunden oder Geschwüren weist eine erste Öffnung zum Aufsetzen auf einen Körperteil eines Patienten und eine zweite Öffnung zum Einschieben eines Handstücks zur Wund- oder Geschwürbehandlung auf. Weiter umfasst die erfindungsgemäße Schutzabdeckung einen flüssigkeitsdichten und flexiblen Hohlkörper, der im Inneren eine Behandlungskammer mit einer sich an eine Bewegung des Handstücks anpassenden Form definiert, wobei der Hohlkörper mit mindestens einem Versteifungselement versehen ist, um die erste Öffnung während der Behandlung in einem geöffneten Zustand zu halten.

Dank der flexiblen Ausführung des Hohlkörpers ist dieser beliebig biegbar und längenveränderbar. Infolgedessen kann die Form der im Inneren des Hohlkörpers gebildeten Behandlungskammer, d.h. der Kammer, die direkt oberhalb an die zu behandelnde Wunde anschließt und das während der Wundbehandlung entstehende Aerosol aufnimmt, an eine beliebige Bewegung des den Spülflüssigkeitsstrahl abgebenden Handstücks angepasst werden. Insbesondere kann das Handstück vom Arzt oder einer Wundfachperson in beliebigem Abstand und beliebigem Winkel zur zu behandelnden Wunde positioniert werden, ohne dass hierdurch die Flüssigkeitsdichtigkeit der Schutzabdeckung beeinträchtigt wird. Aufgrund des zusätzlich im Hohlkörper vorgesehenen Versteifungselements bleibt jedoch die erste, auf dem Körperteil des Patienten zu platzierende Öffnung der Schutzabdeckung bei jeder Bewegung des Handstücks immer in einem geöffneten Zustand. Somit kann diese erste Öffnung mit ihrem Rand bündig auf die Haut des Patienten gesetzt werden, um die zu behandelnde Wunde hermetisch zu umschließen.

Das Versteifungselement kann sich insbesondere nur über einen Teil der Länge der Schutzabdeckung erstrecken, sodass die erste Öffnung nicht unmittelbar an der Unterseite der Schutzabdeckung, sondern hierzu nach oben versetzt ausgebildet ist. Somit ist unterhalb der ersten Öffnung noch ein zusätzlicher unversteifter Materialabschnitt (z. B. Vlies) vorhanden, welcher unter den Körperteil (z. B. die Extremität) des Patienten geklemmt werden kann, um die erste Öffnung dicht abzuschließen.

Zudem muss vorteilhafterweise zur Wundbehandlung lediglich das Handstück des Wundreinigungsgeräts durch die zweite Öffnung der Schutzabdeckung eingeschoben werden, wobei jedoch die das Handstück führende Hand des Arztes oder der sonstigen Wundfachperson außerhalb der Behandlungskammer verbleibt. Hierdurch wird ein potentiell infektiöser Kontakt mit dem Aerosol vermieden.

Eine vorteilhafte Ausgestaltung der Erfindung sieht vor, dass zumindest eine innere, der Behandlungskammer zugewandte Schicht des Hohlkörpers aus einem saugfähigen Material, insbesondere aus einem porösen saufähigen Material besteht zur Aufnahme von Flüssigkeit aus dem während der Behandlung in der Behandlungskammer entstehenden Aerosol.

Die im Aerosol enthaltene Flüssigkeit wird von der saugfähigen Innenschicht aufgenommen und in dieser festgehalten, sodass nur noch relativ wenig oder gar keine Flüssigkeit mehr bis zur Außenschicht bzw. den sonstigen Flächen der Schutzabdeckung durchdringen kann, mit dem vorteilhaften Ergebnis, dass die eventuell in dieser Flüssigkeit enthaltenen pathogenen Krankheitserreger (wie Bakterien, Viren und Pilze) sicher am Entweichen gehindert werden. Die Dicke bzw. Saugfähigkeit der saugfähigen Schicht wird derart bemessen, dass das während der üblichen Wundbehandlungsdauer in das Aerosol abgegebene Gesamtflüssigkeitsvolumen zuverlässig von der saugfähigen Schicht aufgesaugt wird und ohne dass dabei die strukturelle Integrität der saugfähigen Schicht verloren geht.

In weiterer vorteilhafter Ausgestaltung ist vorgesehen, dass zumindest eine äußere, der Umgebung zugewandte Schicht des Hohlkörpers aus einem flüssigkeitsdichten Material, insbesondere aus einem flüssigkeitsdichten Kunststoff, besteht zur Verhinderung eines Austretens von kontaminierter Flüssigkeit in die Umgebung.

Somit kann auch die eventuell durch die saugfähige Innenschicht hindurchtretende Flüssigkeit sicher von der flüssigkeitsdichten Außenschicht (z. B. von einer Polyethylenfolie) zurückgehalten und an einem unkontrollierten Entweichen in die Umgebung gehindert werden. Es versteht sich, dass der Hohlkörper auch einschichtig aus einem Material ausgebildet sein kann, der beide vorgenannten Anforderungen (nämlich Saugfähigkeit und Flüssigkeitsdichtigkeit) in sich vereint, wobei hier insbesondere textile Barrierematerialien, wie bspw. Mikrofasergewebe zu nennen wären.

Nach einer besonders vorteilhaften Ausgestaltung der Erfindung ist der Hohlkörper trichterförmig ausgebildet, wobei seine größere Öffnung die erste Öffnung bildet und seine kleinere Öffnung mit einer transparenten Scheibe zum visuellen Kontrollieren der Behandlungsstelle abgedeckt ist.

Der Durchmesser des trichterförmigen Hohlkörpers vergrößert sich patientenwärts, wobei der Hohlkörper in der ersten, die Wunde oder das Geschwür umgebenden Öffnung seinen größten Durchmesser erreicht, um hier das gesamte Behandlungsfeld sicher abzudichten. In der gegenüberliegenden patientenabgewandten Öffnung des Hohlkörpers ist eine als Sichtfenster fungierende transparente Scheibe eingesetzt, durch welche die behandelnde Person freie Sicht auf das Behandlungsfeld hat.

Gemäß einer weiteren Ausführungsform der Erfindung erstreckt sich das Versteifungselement um die Längsachse des trichterförmigen Hohlkörpers über zumindest einen Teil der Länge seines Mantels schraubenlinienförmig oder kreisförmig.

Die schraubenlinienförmige oder kreisförmige Gestalt des Versteifungselements bewirkt insbesondere eine Verstärkung in radialer Richtung, um die beiden Öffnungen des trichterförmigen Hohlkörpers stabil in einem offenen Zustand zu halten, sodass die untere, durchmessergrößere Öffnung zum Umgeben des Behandlungsfelds und die obere, durchmesserkleinere Öffnung zur Sicht auf das Behandlungsfeld dienen können. Die Länge des schraubenlinienförmigen Versteifungselements ist vorzugsweise so bemessen, dass im unteren, patientenzugewandten Bereich des Hohlkörpers ein versteifungsfreier Überstand an Hohlkörpermaterial verbleibt, der lose auf die Patientenhaut abgelegt und ggf. zusätzlich aufgeklebt werden kann, um das Behandlungsfeld flüssigkeitsdicht abzuschließen.

Ferner ist in vorteilhafter Ausgestaltung der Erfindung vorgesehen, dass das Versteifungselement aus einem elastisch federnden Material, insbesondere aus einem elastisch federnden Kunststoff, besteht, dessen Elastizität eine Kompression und Expansion des trichterförmigen Hohlkörpers in Richtung seiner Längsachse während der Behandlung ermöglicht.

Im Gegensatz zu einer starren Spritzschutzblende erlaubt die erfindungsgemäße Schutzabdeckung, den Abstand des den Flüssigkeitsstrahl abgebenden Handstücks zum Behandlungsfeld je nach dem vorliegenden Wundbild zu variieren. Das Versteifungselement verhält sich dabei ähnlich wie eine niedrigsteife Druckfeder, die nach allen Seiten biegbar und über ihre Länge komprimierbar bzw. expandierbar ist.

Entsprechend einer besonders zweckmäßigen Weiterbildung der Erfindung ist der trichterförmige Hohlkörper aus einer ein- oder mehrschichtig ausgebildeten Materialbahn geformt, deren Enden durch eine Naht miteinander verbunden sind, wobei die Naht insbesondere längs einer Mantellinie des trichterförmigen Hohlkörpers verläuft.

Das Herstellen des Hohlkörpers aus einer einzigen, trichterförmig zusammengerollten Materialbahn erfordert lediglich das Anbringen einer Längsnaht, um die beiden aneinanderstoßenden oder überlappenden Materialbahnenden miteinander zu verbinden. Dies kann fertigungstechnisch besonders einfach und folglich mit geringen Kosten realisiert werden.

In weiterer Ausgestaltung dieser zweckmäßigen Weiterbildung ist das Versteifungselement in die Materialbahn des trichterförmigen Hohlkörpers integriert, insbesondere in die Materialbahn eingeklebt und/oder eingeschweißt und/oder eingenäht.

Das Einnähen unter Verwendung einer Nähmaschine ist vorliegend besonders vorteilhaft, da hierdurch auch artfremde Materialien dauerhaft miteinander verbunden werden können. So kann das Verstärkungselement aus einem für den jeweiligen Anwendungsfall geeigneten Material, z. B. aus einem Kunststoff, insbesondere Kunststofffaser, und/oder aus einem Stoffmaterial und/oder aus einem Folienmaterial bestehen, wobei jedes dieser Materialien problemlos mit der Materialbahn des Hohlkörpers vernäht werden kann.

Das Versteifungselement kann zwischen einer inneren und äußeren Schicht der Materialbahn des trichterförmigen Hohlkörpers angeordnet sein, wodurch eine zuverlässige, ortsfeste und vor äußeren Einflüssen geschützte Positionierung des Versteifungselements erhalten wird.

Ein Ausführungsbeispiel der Erfindung ist in der Zeichnung dargestellt und wird im Folgenden näher beschrieben. Dabei zeigen:
- Fig. 1: eine perspektivische Gesamtansicht eines bevorzugten Ausführungsbeispiels einer Schutzabdeckung nach der Erfindung;
- Fig. 2: eine Seitenansicht auf die Schutzabdeckung der Fig. 1;
- Fig. 3: eine Draufsicht auf die Schutzabdeckung der Fig. 1;
- Fig. 4: eine vergrößerte Draufsicht auf die Schutzabdeckung im Bereich "X" der Fig. 3; und
- Fig. 5: eine vergrößerte Draufsicht auf die Schutzabdeckung im Bereich "Y" der Fig. 4.

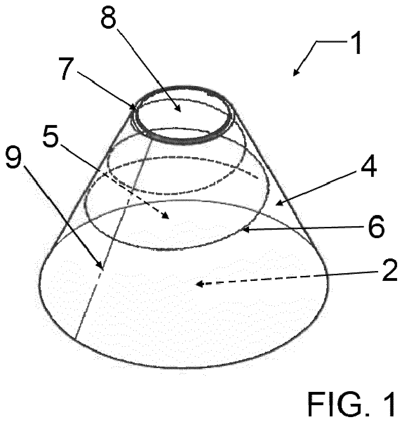

Die in Fig. 1, 2 und 3 jeweils in Gesamtansicht dargestellte erfindungsgemäße Schutzabdeckung 1 besitzt in ihrem undeformierten Ausgangszustand eine der Form einer Qualle ähnelnde Gestalt. Den Kernbestandteil der Schutzabdeckung 1 bildet dabei ein axialsymmetrischer trichterförmiger Hohlkörper 4, der in Richtung seiner Längsachse A zwei Öffnungen 2, 7 aufweist, die ihrerseits axial und diametral zueinander angeordnet sind, wobei diese Öffnungen 2, 7 von unterschiedlicher Größe sind.

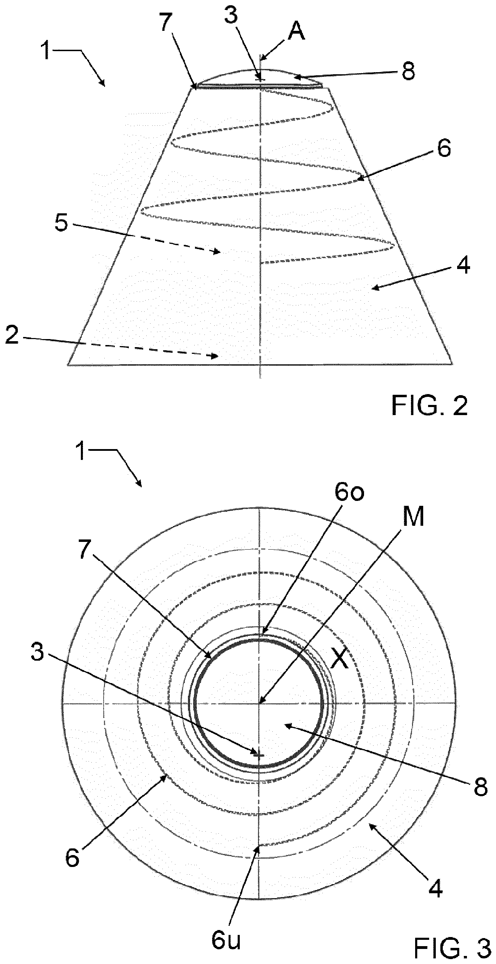

Die größere, in den Fig. 1 und 2 jeweils nach unten weisende Öffnung 2 wird im späteren Gebrauch auf einen Körperteil, in der Regel eine Extremität, eines Patienten aufgesetzt, wobei die Ränder der Öffnung 2 ein Behandlungsfeld begrenzen, in dem sich eine mit einem Spülflüssigkeitsstrahl zu behandelnde Wunde oder eine sonstige Hauterkrankung (z. B. Geschwür) eines Patienten befindet.

Der Hohlkörper 4 umgibt einen kegelstumpfförmigen Innenraum, der im späteren Gebrauch als Behandlungskammer 5 fungiert, in welcher das (nicht gezeigte) Kopfende eines Handstücks eingeführt ist, von welchem wiederum zur Wundbehandlung (Wundspülung und ggf. Wunddebridement) ein Spülflüssigkeitsstrahl abgegeben wird. Das Handstück ist hierzu außerhalb der Behandlungskammer 5 über einen Schlauch mit einem Spülflüssigkeitsbehälter zur Zufuhr einer unter Druck stehenden Spülflüssigkeit (z. B. steriles Wasser oder Kochsalzlösung) verbunden.

Bei dem Handstück kann es sich jedoch um jedes beliebige zur lokalen Wundbehandlung von Hand bewegbare Teil eines Wundbehandlungsgeräts handeln. Beispielsweise kann vom Handstück anstatt eines Spülflüssigkeitsstrahls zur Wundbehandlung auch ein Plasma oder Ultraschallwellen abgegeben werden. Entscheidend ist allein, dass während der Wundbehandlung mittels des Handstücks ein gesundheitsschädliches Aerosol entsteht, das der Abschirmung bedarf.

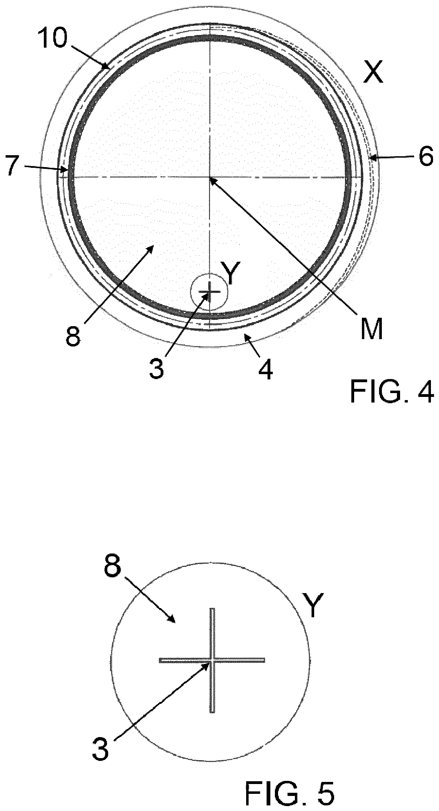

Um die Behandlungskammer 5 zum einen flüssigkeitsdicht nach oben hin abzuschließen und zum anderen der behandelnden Person eine freie Sicht auf das von der gegenüberliegenden unteren Öffnung 2 berandete Behandlungsfeld zu gewähren, ist die in den Fig. 1 und 2 jeweils nach oben weisende, kleinere Öffnung 7 des trichterförmigen Hohlkörpers 4 von einer kreisförmigen starren Scheibe 8 aus einem transparenten Material, bspw. aus einem transparenten Kunststoff wie Plexiglas, abgedeckt. Die Scheibe 8 ist - wie in der vergrößerten Draufsicht gemäß Fig. 4 gut zu erkennen - mittels einer kreisförmig umlaufenden Naht 10, die bspw. maschinell durch eine Nähmaschine produziert werden kann, mit dem oberen Öffnungsrand des Hohlkörpers 4 verbunden. Alternativ wäre es jedoch auch vorstellbar, die Scheibe 8 nur lose auf die kleinere Öffnung 7 aufzulegen. In einem solchen Fall könnte die Scheibe 8 als Einwegartikel nach jeder Wundbehandlung separat vom Hohlkörper 4 entsorgt werden.

Wie aus Fig. 2 hervorgeht, besitzt die Scheibe 8 eine schüsselförmige, nach außen hin konvex gekrümmte Gestalt, wobei die Scheibenkrümmung einen Lupeneffekt bewirken kann, um die zu behandelnde Wunde genauer einsehen zu können. Außerdem kann durch eine solche Scheibenkrümmung eine sichtbehindernde Ansammlung von Flüssigkeitstropfen verhindert werden.

Wie insbesondere aus den vergrößerten Ansichten gemäß Fig. 4 und 5 erkennbar, weist die transparente Scheibe 8 exzentrisch, also vom mit der Längsachse A des Hohlkörpers 4 fluchtenden Mittelpunkt M der Scheibe 8 zum Rand hin versetzt, eine Öffnung in Form eines Kreuzschlitzes 3 auf. Dieser Kreuzschlitz 3 dient zum Einschieben eines Handstücks zur Wundbehandlung (z. B. eines einen Spülflüssigkeitsstrahl, Plasma oder Ultraschallwellen abgebenden Handstücks) und ist in der Draufsicht gemäß Fig. 4 mit einer senkrecht unterhalb des Scheibenmittelpunkts M befindlichen Anordnung dargestellt. Im späteren Gebrauch kann die Schutzabdeckung 1 jedoch so gedreht werden, dass der Kreuzschlitz 3 für die behandelnde Person eine zur Einführung und Handhabung des Handstücks angenehme Position einnimmt. So wird ein Rechtshänder im Regelfall die Schutzabdeckung 1 derart um ihre Längsachse A verdrehen, da sich der Kreuzschlitz 3 aus seiner Sicht rechts der Längsachse A und des Scheibenmittelpunkts M befindet.

Die Schlitzbreiten des Kreuzschlitzes 3 betragen bevorzugt ca. 0,5 mm. Am Kreuzungspunkt der beiden Schlitze wird das Kopfende des Handstücks eingesteckt, wodurch sich der Kreuzschlitz 3 zunächst elastisch aufweitet. Ist das Kopfende in den Behandlungsraum 5 eingeführt, so legt sich der Kreuzschlitz 3 wieder flüssigkeitsdicht an den Außenumfang des Handstücks an, sodass keine kontaminierte Flüssigkeit zwischen dem Kreuzschlitz 3 und dem Handstück austreten kann.

Die behandelnde Person muss das Handstück lediglich so weit durch den Kreuzschlitz 3 der Scheibe 8 vorschieben, bis die am Kopfende des Handstücks angeordnete Düse innerhalb der Behandlungskammer 5 (unterhalb der Scheibe 8) positioniert ist. Der übrige Teil des Handstücks verbleibt jedoch außerhalb der Behandlungskammer 5 (oberhalb der Scheibe 8), um z. B. mittels eines Handgriffs durch die Hand der behandelnden Person zwecks Durchführung der Wundbehandlung bewegt zu werden. Da das Führen des Spülflüssigkeitsstrahls somit durch einen oberhalb der Scheibe 8 angeordneten Abschnitt des Handstücks erfolgt und da gleichzeitig durch die Scheibe 8 freie Sicht auf den unterhalb der Scheibe 8 erzeugten Flüssigkeitsstrahl und die Wunde gegeben ist, muss die behandelnde Person mit ihren Händen nicht länger in die aerosolbelastete Behandlungskammer 5 eingreifen. Hierdurch wird das Infektionsrisiko deutlich herabgesetzt. Dieser vorteilhafte Effekt ist auch dann erzielbar, wenn die Öffnung (Kreuzschlitz 3) zum Einschieben des Handstücks nicht - wie in Fig. 3 gezeigt - in der Scheibe 8, sondern stattdessen im Hohlkörper 4 ausgebildet ist. In einem solchen Fall würde die Scheibe 8 als reines Sichtfenster dienen.

In Ergänzung zum gezeigten Ausführungsbeispiel ist es jedoch auch denkbar, seitlich im Hohlkörper 4 Handlöcher vorzusehen, durch die eine behandschuhte Hand im Bedarfsfall in die Behandlungskammer 5 eingreifen kann, um z.B. die während der Wundbehandlung abfließende Behandlungsflüssigkeit mit Kompressen oder Tupfern aufzufangen. Diese Handlöcher können zusätzlich mit integrierten Handschuhen ausgestattet sein. Wichtig ist dabei, dass weder im Bereich der Handlöcher noch im Bereich der evtl. dort integrierten Handschuhe Spülflüssigkeit aus der Behandlungskammer 5 entweichen kann. Außerdem können seitlich am Hohlkörper 4 zusätzliche Laschen angeformt sein, um die Schutzabdeckung 1 als Ganzes anzuheben oder ggf. im ungebrauchten Zustand aufzuhängen.

Ein großes Gefährdungspotential geht von den Aerosolen aus, die während der Wundbehandlung aufgrund des hohen Aufpralldrucks des Spülflüssigkeitsstrahls oberhalb der Wundbehandlungsstelle entstehen. Bedingt durch die bakterielle, virale, fungale oder parasitäre Belastung der meisten Wunden sind nämlich auch diese Aerosole zumeist hochkontagiös. Außerdem beeinträchtigt das entstehende Aerosol die freie Sicht auf das Behandlungsfeld, wodurch es der behandelnden Person erschwert wird, den Wundbehandlungsfortschritt genau zu erfassen.

Demzufolge wird an das Material des Hohlkörpers 4 zum einen die Anforderung gestellt, dass es eine ausreichende Saugfähigkeit aufweist, um aus dem während der Wundbehandlung in der Behandlungskammer 5 entstehenden Aerosol die Flüssigkeitstropfen aufzunehmen und festzuhalten. Dabei ist es ausreichend, lediglich die der Behandlungskammer 5 zugewandte Innenschicht des Hohlkörpers 4 mit einem saugfähigen Material auszustatten. Als saugfähiges Material kann ein Papier, ein Textil oder ein Vlies zur Anwendung kommen. Diese Materialien haben die vorteilhafte Eigenschaft, dass eine Sättigung des Aufnahmevermögens auch bei langer Behandlungsdauer nicht erreicht wird und dass somit die Adsorption durch Oberflächenbenetzung während der gesamten Behandlungszeit wirken kann. Diese Adsorption wirkt als treibende Kraft, welche die flüssigkeitsgebundenen pathogenen Krankheitserreger (wie z. B. Bakterien) an den Fasern des Papiers, Textils oder Vlieses bindet. Die kleinen, mit Erregern beladenen Tröpfchen werden dabei sehr schnell vom Papier, Textil oder Vlies aufgenommen und gefangen. Mit zunehmender, im Papier, Textil oder Vlies gebundener Flüssigkeitsmenge wird die flüssigkeitsgetränkte Oberfläche erweitert und der Adsorptionseffekt kann sogar beschleunigt werden.

Neben Saugfähigkeit wird vom Material des Hohlkörpers 4 zum anderen Flüssigkeitsdichtigkeit gefordert, um die während der Wundbehandlung in die Behandlungskammer 5 geschleuderten, mit Wundsekret vermischten hochkontagiösen Körper- und Spülflüssigkeitstropfen sicher an einem Austreten in die Umgebung zu hindern und somit eine Kontamination der Umgebung zu vermeiden. Hierfür ist es ausreichend, wenn wenigstens eine Außenschicht, d.h. eine von der Behandlungskammer 5 abgewandte Schicht, des Hohlkörpers 4 aus einem flüssigkeitsdichten Material, z. B. einer flüssigkeitsdichten Kunststofffolie, besteht.

Ein bevorzugtes mehrschichtiges Material für den Hohlkörper 4, das die vorbeschriebenen Eigenschaften hinsichtlich Saugfähigkeit und Flüssigkeitsdichtigkeit erfüllt, weist als eine Außenschicht eine Polyethylenfolie und als eine Innenschicht eine oder zwei Lagen Zellstoff auf.

Bei Verwendung des vorgenannten Materials besteht jedoch das grundsätzliche Problem, dass dieses keine oder nur eine geringe Eigenstabilität besitzt. Wie eine üblicherweise zur Patientenabdeckung verwendete Einwegschutzfolie würde ein solches Material in sich zusammenfallen, falls keine zusätzlichen Versteifungsmaßnamen getroffen werden. Insbesondere muss gewährleistet sein, dass die zum Umgeben der Wunde auf den Patientenkörper aufgesetzte untere Öffnung 2 jederzeit in einem geöffneten Zustand gehalten wird. Nur so kann sichergestellt werden, dass das Hohlkörpermaterial nicht in die Wunde des Patienten oder in das Sichtfeld der behandelnden Person gelangen kann.

Um - wie in Fig. 1 und 2 gezeigt - ein stabiles Aufstellen der Schutzabdeckung 1 mit einer dauerhaft in geöffneter Position gehaltenen unteren Öffnung 2 zu erreichen, ist ein der Deformation des Hohlkörpers 4 in radialer und axialer Richtung entgegenwirkendes Versteifungselement 6 vorgesehen, welches gemäß den in Fig. 1 und 2 gezeigten Gesamtansichten als ein sich schraubenlinienförmig mit zweieinhalb Windungen um die Längsachse A erstreckender Kunststoffdraht (mit einem Durchmesser von z. B. 2 mm) ausgebildet ist.

Das schraubenlinienförmige Versteifungselement 6 ist in das Material des Hohlkörpers 4 integriert, wobei diese Integration z. B. durch Einnähen des Versteifungselements 6 erfolgen kann. Diese Integration kann in fester oder lösbarer Art erfolgen. Im letztgenannten Fall kann das Versteifungselement 6 z. B. lediglich in das Material des Hohlkörpers 4 eingeschoben oder eingeklickt sein. Durch Nähen lassen sich artfremde Materialien problemlos miteinander verbinden, weshalb das Versteifungselement 6 statt aus einem Kunststoff auch aus einem beliebig anderen Material mit versteifender Wirkung, z. B. einem Metall, bestehen kann. Auch muss das Versteifungselement 6 nicht notwendigerweise in Drahtform, sondern kann auch in jeder anderen in den Hohlkörper 4 integrierbaren Form, z.B. in Folienform, vorliegen. Bei einem mehrschichtig aufgebauten Hohlkörper 4 ist das Versteifungselement 6 bevorzugt zwischen den Schichten angeordnet. Solch eine eingebettete Anordnung hat den Vorteil, dass das Versteifungselement 6 vor äußeren Einflüssen, z. B. vor dem während der Wundbehandlung entstehenden Aerosol, geschützt ist und dass zudem eine besonders positionsstabile Festlegung des Versteifungselements 6 im Hohlkörper 4 erreicht wird.

In der Draufsicht gemäß Fig. 3 verläuft das Versteifungselement 6 spiralförmig um den in Verlängerung der Längsachse A angeordneten Mittelpunkt M der Scheibe 8. Dabei sind der in Längsrichtung gesehen obere Endpunkt 6o des Versteifungselements 6 und der in Längsrichtung gesehen untere Endpunkt 6u der Versteifungselements 6 bezogen auf den Mittelpunkt M der Scheibe 8 diametral gegenüberliegend angeordnet, wobei sich der Kreuzschlitz 3 zum Einschieben des den Flüssigkeitsstrahl abgebenden Handstücks auf der Verbindungslinie dieser beiden Endpunkte 6o, 6u befindet.

Trotz des Vorsehens eines Versteifungselements 6 bleibt gleichwohl eine Flexibilität des Hohlkörpers 4 erhalten, die es gestattet, die Form der im Inneren des Hohlkörpers 4 definierten Behandlungskammer 5 an eine beliebige Bewegung des Handstücks anzupassen. Das Versteifungselement 6 weist ähnliche Deformationseigenschaften wie eine niedrigsteife Druckfeder mit hoher Steigung auf. Dementsprechend kann der Hohlkörper 4 mit dem integrierten Versteifungselement 6 weiterhin zu allen Seiten hin frei gebogen und auch in Längsrichtung komprimiert bzw. expandiert werden. Diese Deformationseigenschaften erlauben es, während der Wundbehandlung das Handstück in einen beliebigen Winkel und/oder in einen beliebigen Abstand zur zu behandelnden Wunde zu positionieren.

Die behandelnde Person wird hierzu während der Wundbehandlung über eine freie Hand und/oder unmittelbar über das in den Kreuzschlitz 3 eingeführte Handstück auf den Hohlkörper 4 eine deformierende Kraft ausüben, um die Form des Hohlkörpers 4 und der von ihr definierten Behandlungskammer 5 an eine neue Position des Handstücks anzupassen. Ungeachtet davon bleibt jedoch die Flüssigkeitsdichtigkeit der Behandlungskammer 5 jederzeit gewahrt. Die Behandlungskammer 5 ist nämlich unabhängig von der gerade angenommenen Form nach oben hin immer durch die aufgesetzte Scheibe 8 und zu allen Seiten hin immer durch den Mantel des Hohlkörpers 4 flüssigkeitsdicht abgeschlossen.

Die durch das integrierte Versteifungselement 6 in radialer und axialer Richtung des Hohlkörpers 4 erzielte Versteifungswirkung führt aber vorteilhafterweise dazu, dass der Hohlkörper 4 unabhängig von seiner aktuellen, an die Bewegung des Handstücks angepassten Form jederzeit eine stabil geöffnete untere Öffnung 2 aufweist. Das Versteifungselement 6 öffnet dabei also die Behandlungskammer 5. Im Gebrauch ist es ausreichend, die Schutzabdeckung 1 mit einer freien Hand, z. B. am Rand der Scheibe 8, festzuhalten. Es kann jedoch auch ganz auf ein manuelles Festhalten der Schutzabdeckung 1 verzichtet werden, da diese bereits von dem im Kreuzschlitz 3 eingeschobenen Handstück fixiert ist. Vorteilhafterweise stehen somit beide Hände des Arztes oder der sonstigen Wundfachperson für die Wundbehandlung zur Verfügung.

Durch die in Richtung der Längsachse A bestehende Kompressibilität des Hohlkörpers 4 ist es möglich, die Schutzabdeckung 1 durch leichten manuellen Druck bündig auf einen beliebigen Körperteil eines Patienten aufzusetzen. Damit ist die unterseitige Form der Schutzabdeckung 1 optimal an die Form des jeweiligen Körperteils angepasst, wodurch das Risiko einer Undichtigkeit zwischen dem Patientenkörper und der Schutzabdeckung 1 auf ein Minimum reduziert ist. Zudem kann aufgrund der Kompressibilität des Hohlkörpers 4 die Schutzabdeckung 1 zu Versand- und Lagerzwecken nahezu platt zusammengedrückt werden, um ggf. in eine entsprechende Verpackungshülle eingelegt zu werden.

Der Hohlkörper 4 ist in fertigungstechnisch besonders einfacher und somit kostengünstiger Weise aufgebaut, da er lediglich aus einer einzigen Materialbahn besteht. Zur Bildung des Hohlkörpers 4 wird eine bogenförmig gekrümmte Materialbahn derart zusammengerollt, dass die beiden gegenüberliegenden Materialbahnenden überlappend aufeinander zu liegen kommen und anschließend mit einem Faden durchgenäht werden. Eine solche Überlappnaht 9 ist grundsätzlich haltbarer als Stoß-an-Stoß-Konstruktionen und auch meist einfacher herstellbar. Die Überlappnaht 9 ist in der fertigen Schutzabdeckung 1 innenseitig zur Behandlungskammer 5 hin angeordnet, um die Dichtigkeit und den visuellen Gesamteindruck des Hohlkörpers 4 nicht zu beeinträchtigen. Im undeformierten Ausgangszustand der Schutzabdeckung 1 gemäß Fig. 1 verläuft die Überlappnaht 9 längs einer Mantellinie des trichterförmigen Hohlkörpers 4.

Das schraubenlinienförmige Versteifungselement 6 erstreckt sich - in Richtung der Längsachse A gesehen - nicht über die Gesamtlänge des Hohlkörpers 4. Zwar beginnt das Versteifungselement 6 nahezu bündig an dem von der Scheibe 8 abgedeckten oberen Rand des Hohlkörpers 4, endet jedoch in deutlichem Abstand zum unteren Rand des Hohlkörpers 4. Die in Richtung der Längsachse A gemessene Länge des Versteifungselements 6 nimmt im Verhältnis zur Gesamtlänge des Hohlkörpers 4 einen Wert von ca. 1/2 bis 3/4, bevorzugt von ca. 2/3, an. Demzufolge besteht ein unterer Längsabschnitt des Hohlkörpers 4 nur aus der unverstärkten Materialbahn (ohne integriertes Versteifungselement 6). Dieser untere Längsabschnitt kann im späteren Gebrauch lose auf den Patientenkörper abgelegt und ggf. zusätzlich aufgeklebt werden, um einen nach unten hin dichten Abschluss zwischen Patientenkörper und Schutzabdeckung 1 herzustellen.

Die erfindungsgemäße Schutzabdeckung 1 kann in jeder Vorrichtung zum Spülen von Wunden oder Geschwüren vorteilhaft zur Anwendung gelangen. Insbesondere kann sie Teil einer Vorrichtung zum Debridieren von abgestorbenem Körpergewebe mittels eines Mikrowasserstrahls sein. Bei einer solchen Vorrichtung wird als Handgerät eine von Hand zu führende Lanze verwendet, wobei diese Lanze so weit durch den Kreuzschlitz 3 der Schutzabdeckung 1 eingeschoben wird, dass sich die am freien Ende der Lanze angeordnete Düse zur Erzeugung des Mikrowasserstrahls innerhalb der Behandlungskammer 5 befindet. Durch die Verwendung der erfindungsgemäßen Schutzabdeckung 1 wird eine hohe Sicherheit vor Kontamination der Umgebung mit Körper- und/oder Spülflüssigkeit während des Wunddebridements erreicht, ohne dass hierbei jedoch die Bewegungsfreiheit des Handstücks und die Sicht der behandelnden Person auf die zu debridierende Wunde und den Mikrowasserstrahl eingeschränkt werden.

### Bezugszeichenliste

- 1: Schutzabdeckung
- 2: erste, untere Öffnung
- 3: zweite Öffnung / Kreuzschlitz
- 4: Hohlkörper
- 5: Behandlungskammer
- 6: Versteifungselement
- 6o: oberer Endpunkt des Versteifungselements
- 6o: unterer Endpunkt des Versteifungselements
- 7: kleinere Öffnung
- 8: Scheibe
- 9: (Überlapp)Naht des Hohlkörpers
- 10: Naht zwischen Scheibe und Hohlkörper)
- A: Längsachse des Hohlkörpers
- M: Mittelpunkt der Scheibe

## Patentansprüche

1. Schutzabdeckung (1) zum Schutz vor Kontamination mit Körper- und/oder Spülflüssigkeit bei der Behandlung von Wunden oder Geschwüren,
wobei die Schutzabdeckung (1) eine erste Öffnung (2) zum Aufsetzen auf einen Körperteil eines Patienten und eine zweite Öffnung (3) zum Einschieben eines Handstücks zur Wund- oder Geschwürbehandlung aufweist,
wobei die Schutzabdeckung (1) einen flüssigkeitsdichten und flexiblen Hohlkörper (4) umfasst, der im Inneren eine Behandlungskammer (5) mit einer sich an eine Bewegung des Handstücks anpassenden Form definiert,
wobei der Hohlkörper (4) trichterförmig ausgebildet ist und seine größere Öffnung die erste Öffnung (2) bildet und
wobei der Hohlkörper (4) mit mindestens einem Versteifungselement (6) versehen ist, um die erste Öffnung (2) während der Behandlung in einem geöffneten Zustand zu halten,
**dadurch gekennzeichnet, dass**
das Versteifungselement (6) sich um die Längsachse (A) des trichterförmigen Hohlkörpers (4) über zumindest einen Teil der Länge seines Mantels schraubenlinienförmig erstreckt.

2. Schutzabdeckung nach Anspruch 1, **dadurch gekennzeichnet, dass** zumindest eine innere, der Behandlungskammer (5) zugewandte Schicht des Hohlkörpers (4) aus einem saugfähigen Material, insbesondere aus einem porösen saugfähigen Material, besteht zur Aufnahme von Flüssigkeit aus dem während der Behandlung in der Behandlungskammer (5) entstehenden Aerosol.

3. Schutzabdeckung nach Anspruch 1 oder 2, **dadurch gekennzeichnet, dass** zumindest eine äußere, der Umgebung zugewandte Schicht des Hohlkörpers (4) aus einem flüssigkeitsdichten Material, insbesondere aus einem flüssigkeitsdichten Kunststoff, besteht zur Verhinderung eines Austretens von kontaminierter Flüssigkeit in die Umgebung.

4. Schutzabdeckung nach zumindest einem der vorhergehenden Ansprüche 1 bis 3, **dadurch gekennzeichnet, dass** die kleinere Öffnung (7) des Hohlkörpers (4) mit einer transparenten Scheibe (8) zum visuellen Kontrollieren der Behandlungsstelle abgedeckt ist.

5. Schutzabdeckung nach zumindest einem der vorhergehenden Ansprüche 1 bis 4, **dadurch gekennzeichnet, dass** das Versteifungselement (6) aus einem elastisch federnden Material, insbesondere aus einem elastisch federnden Kunststoff, besteht, dessen Elastizität eine Kompression und Expansion des trichterförmigen Hohlkörpers (4) in Richtung seiner Längsachse (A) während der Behandlung ermöglicht.

6. Schutzabdeckung nach zumindest einem der vorhergehenden Ansprüche 1 bis 5, **dadurch gekennzeichnet, dass** der trichterförmige Hohlkörper (4) aus einer ein- oder mehrschichtig ausgebildeten Materialbahn geformt ist, deren Enden durch eine Naht (9) miteinander verbunden sind, wobei die Naht (9) längs einer Mantellinie des trichterförmigen Hohlkörpers (4) verläuft.

7. Schutzabdeckung nach Anspruch 6, **dadurch gekennzeichnet, dass** das Versteifungselement (6) in die Materialbahn des trichterförmigen Hohlkörpers (4) integriert ist, insbesondere in die Materialbahn eingeklebt und/oder eingeschweißt und/oder eingenäht ist.

8. Schutzabdeckung nach Anspruch 6 oder 7, **dadurch gekennzeichnet, dass** das Versteifungselement (6) zwischen einer inneren und äußeren Schicht der Materialbahn des trichterförmigen Hohlkörpers (4) angeordnet ist.

9. Schutzabdeckung nach zumindest einem der vorhergehenden Ansprüche 4 bis 8, **dadurch gekennzeichnet, dass** die transparente Scheibe (8) lose aufgelegt ist, um als Einwegartikel nach abgeschlossener Behandlung entsorgt zu werden.

10. Schutzabdeckung nach zumindest einem der vorhergehenden Ansprüche 4 bis 8, **dadurch gekennzeichnet, dass** die transparente Scheibe (8) durch eine kreisförmig umlaufende Naht (10) mit dem trichterförmigen Hohlkörper (4) verbunden ist.

11. Schutzabdeckung nach zumindest einem der vorhergehenden Ansprüche 4 bis 10, **dadurch gekennzeichnet, dass** die transparente Scheibe (8) als konvex gekrümmte Scheibe ausgeführt ist, deren Krümmung von der Behandlungskammer (5) weg nach außen gerichtet ist.

12. Schutzabdeckung nach zumindest einem der vorhergehenden Ansprüche 4 bis 11, **dadurch gekennzeichnet, dass** die transparente Scheibe (8) aus einem starren Material, insbesondere aus einem starren Kunststoff oder Glas besteht, wobei die zweite Öffnung (3) in der transparenten Scheibe (8) vorgesehen ist.

13. Schutzabdeckung nach zumindest einem der vorhergehenden Ansprüche 1 bis 12, **dadurch gekennzeichnet, dass** die zweite Öffnung (3) so ausgebildet ist, dass sie sich beim Einschieben des Handstücks radial aufweitet und sich nach Abschluss des Einschiebens im Wesentlichen flüssigkeitsdicht an den Außenumfang des Handstücks anlegt.

14. Schutzabdeckung nach Anspruch 13, **dadurch gekennzeichnet, dass** die zweite Öffnung (3) als Kreuzschlitz ausgebildet ist.

15. Vorrichtung zum Spülen von Wunden oder Geschwüren, insbesondere Vorrichtung zum Debridieren von abgestorbenem Körpergewebe mittels eines Mikrowasserstrahls, umfassend eine Schutzabdeckung (1) nach zumindest einem der vorhergehenden Ansprüche 1 bis 14 und eine von Hand zu führende Lanze, an deren Ende eine Düse zur Erzeugung eines Spülflüssigkeitsstrahls, insbesondere eines Mikrowasserstrahls, angeordnet ist, wobei die Lanze so weit durch die zweite Öffnung (3) einer Schutzabdeckung (1) nach zumindest einem der vorhergehenden Ansprüche 1 bis 14 eingeschoben ist, dass sich die Düse innerhalb der Behandlungskammer (5) der Schutzabdeckung (1) befindet.

## Claims

1. Protective cover (1) for the protection against contamination from body and/or irrigation fluid during the treatment of wounds or ulcers,
where said protective cover (1) comprises a first opening (2) to be placed on a body part of a patient and a second opening (3) adapted for the insertion of a handpiece for wound or ulcer treatment,
where said protective cover (1) comprises a fluid-tight and flexible hollow body (4) which in the interior defines a treatment chamber (5) having a shape that adapts to the motion of said handpiece,
where said hollow body (4) is formed having a funnel shape and its larger opening forms said first opening (2),
where said hollow body (4) is provided with at least one stiffening element (6) for maintaining said first opening (2) in an open state during treatment, **characterized in that**
said stiffening element (6) extends around the longitudinal axis (A) of said funnel-shaped hollow body (4) in a helical or circular shape over at least part of the length of its shell.

2. Protective cover according to claim 1, **characterized in that** at least one inner layer of said hollow body (4) facing said treatment chamber (5) is made of absorbent material, in particular of porous absorbent material, for receiving fluid from the aerosol produced during the treatment in said treatment chamber (5).

3. Protective cover according to claim 1 or 2, **characterized in that** at least one outer layer of said hollow body (4) facing the environment is made of fluid-tight material, in particular fluid-tight plastic, for preventing the contaminated fluid from escaping into the environment.

4. Protective cover according to at least one of the preceding claims 1 to 3, **characterized in that** said smaller opening (7) of said hollow body (4) is covered with a transparent plate (8) for visually monitoring the treatment area.

5. Protective cover according to at least one of the preceding claims 1 to 4, **characterized in that** said stiffening element (6) is composed of elastically resilient material, in particular of elastically resilient plastic, the elasticity of which during the treatment allows the compression and expansion of said funnel-shaped hollow body (4) in the direction of its longitudinal axis (A).

6. Protective cover according to at least one of the preceding claims 1 to 5, **characterized in that** said funnel-shaped hollow body (4) is formed from a web of material that is composed of one or more layers, the ends of which are connected to each other by a seam (9), where said seam (9) extends in particular along a surface line of said funnel-shaped hollow body (4).

7. Protective cover according to claim 6, **characterized in that** said stiffening element (6) is integrated into said web of material of said funnel-shaped hollow body (4), in particular glued into and/or welded into and/or sewn into said web of material.

8. Protective cover according to claim 6 or 7, **characterized in that** said stiffening element (6) is disposed between an inner and an outer layer of said web of material of said funnel-shaped hollow body (4).

9. Protective cover according to at least one of the preceding claims 4 to 8, **characterized in that** said transparent plate (8) is loosely fitted in order to be disposed of as a disposable item after wound treatment has been completed.

10. Protective cover according to at least one of the preceding claims 4 to 8, **characterized in that** said transparent plate (8) is connected to said funnel-shaped hollow body (4) by a circular circumferential seam (10).

11. Protective cover according to at least one of the preceding claims 4 to 10, **characterized in that** said transparent plate (8) is configured as a convex curved plate, the curvature of which is directed outwardly away from said treatment chamber (5).

12. Protective cover according to at least one of the preceding claims 4 to 11, **characterized in that** said transparent plate (8) is made of rigid material, in particular rigid plastic or glass, where said second opening (3) is provided in said transparent plate (8).

13. Protective cover according to at least one of the preceding claims 1 to 12, **characterized in that** said second opening (3) is configured such that it expands radially upon insertion of said handpiece and after completion of the insertion bears substantially in a fluid-tight manner against the outer perimeter of said handpiece.

14. Protective cover according to claim 13, **characterized in that** said second opening (3) is formed as a cross recess.

15. Device for the irrigation of wounds or ulcers, in particular a device for debriding dead body tissue by way of a micro water jet, comprising a protective cover (1) according to at least one of the preceding claims 1 to 14 and a hand-operated lance, at the end of which a nozzle is disposed for producing an irrigation fluid jet, in particular a micro water jet, where said lance is inserted so far through said second opening (3) of a protective cover (1) according to at least one of the preceding claims 1 to 14 that said nozzle is located within said treatment chamber (5) of said protective cover (1).

## Revendications

1. Revêtement de protection (1) pour protéger contre la contamination par les liquides corporels et/ou d'irrigation lors du traitement de blessures ou d'ulcères,
dans lequel le revêtement de protection (1) comporte une première ouverture (2) pour placer sur une partie du corps d'un patient et une seconde ouverture (3) pour insérer une pièce à main pour le traitement des plaies ou des ulcères,
dans lequel le revêtement de protection (1) comprend un corps creux (4) étanche aux liquides et flexible qui définit intérieurement une chambre de traitement (5) ayant une forme qui s'adapte à un mouvement de la pièce à main,
dans laquelle le corps creux (4) est en forme d'entonnoir et son ouverture plus grande forme la première ouverture (2) et
dans lequel le corps creux (4) est muni d'au moins un élément de raidissement (6) pour maintenir la première ouverture (2) dans un état ouvert pendant le traitement,
**caractérisé en ce que**
l'élément de raidissement (6) s'étend en hélice autour de l'axe longitudinal (A) du corps creux en forme d'entonnoir (4) sur au moins une partie de la longueur de son enveloppe.

2. Revêtement de protection selon la revendication 1, **caractérisé en ce qu'au** moins une couche intérieure du corps creux (4) tournée vers la chambre de traitement (5) est constituée d'un matériau absorbant, en particulier d'un matériau absorbant poreux, pour absorber le liquide de l'aérosol produit dans la chambre de traitement (5) pendant le traitement.

3. Revêtement de protection selon les revendications 1 ou 2, **caractérisé en ce qu'au** moins une couche extérieure du corps creux (4) tournée vers l'environnement est constituée d'un matériau étanche aux liquides, en particulier d'une matière plastique étanche aux liquides, pour empêcher que du liquide contaminé ne s'échappe dans l'environnement.

4. Revêtement de protection selon au moins une des revendications précédentes 1 à 3, **caractérisé en ce que** la plus petite ouverture (7) du corps creux (4) est recouverte d'une vitre transparente (8) pour l'inspection visuelle du site de traitement.

5. Revêtement de protection selon au moins l'une des revendications précédentes 1 à 4, **caractérisé en ce que** l'élément de raidissement (6) est constitué d'un matériau élastique, en particulier d'une matière plastique élastique, dont l'élasticité permet la compression et l'expansion du corps creux (4) en forme d'entonnoir dans la direction de son axe longitudinal (A) pendant le traitement.

6. Revêtement de protection selon au moins l'une des revendications 1 à 5 précédentes, **caractérisé en ce que** le corps creux en forme d'entonnoir (4) est formé d'une bande de matériau monocouche ou multicouche, dont les extrémités sont reliées entre elles par une couture (9), la couture (9) s'étendant le long d'une génératrice du corps creux en forme d'entonnoir (4).

7. Revêtement de protection selon la revendication 6, **caractérisé en ce que** l'élément de raidissement (6) est intégré dans la bande de matériau du corps creux en forme d'entonnoir (4), en particulier est collé et/ou soudé et/ou cousu dans la bande de matériau.

8. Revêtement de protection selon les revendications 6 ou 7, **caractérisé en ce que** l'élément de raidissement (6) est disposé entre une couche intérieure et une couche extérieure de la bande de matériau du corps creux (4) en forme d'entonnoir.

9. Revêtement de protection selon au moins une des revendications 4 à 8 précédentes, **caractérisé en ce que** la vitre transparente (8) est appliquée de manière lâche afin d'être éliminée comme un article jetable une fois le traitement terminé.

10. Revêtement de protection selon au moins une des revendications précédentes 4 à 8, **caractérisé en ce que** la vitre transparente (8) est reliée au corps creux (4) en forme d'entonnoir par une couture circulaire (10).

11. Revêtement de protection selon au moins une des revendications précédentes 4 à 10, **caractérisé en ce que** la vitre transparente (8) est conçue comme une vitre à courbure convexe dont la courbure est dirigée vers l'extérieur à l'opposé de la chambre de traitement (5).

12. Revêtement de protection selon au moins une des revendications précédentes 4 à 11, **caractérisé en ce que** la feuille transparente (8) est faite d'un matériau rigide, en particulier d'un plastique rigide ou de verre, la seconde ouverture (3) étant prévue dans la feuille transparente (8).

13. Revêtement de protection selon au moins l'une des revendications 1 à 12 précédentes, **caractérisé en ce que** la seconde ouverture (3) est conçue de telle sorte qu'elle se dilate radialement lors de l'insertion de la pièce à main et, une fois l'insertion terminée, s'applique contre la circonférence extérieure de la pièce à main d'une manière sensiblement étanche aux liquides.

14. Revêtement de protection selon la revendication 13, **caractérisé en ce que** la seconde ouverture (3) est conçue comme une fente transversale.

15. Dispositif pour le rinçage de plaies ou d'ulcères, en particulier dispositif pour le débridement de tissus de cadavres au moyen d'un microjet d'eau, comprenant un revêtement de protection pur (1) selon au moins une des revendications précédentes 1 à 14 et une lance guidée manuellement, à l'extrémité de laquelle est prévue une buse pour la génération d'un jet de liquide de rinçage, en particulier un microjet d'eau, la lance étant introduite par la seconde ouverture (3) d'un revêtement de protection (1) selon au moins l'une des revendications précédentes 1 à 15, de telle sorte que la buse se trouve à l'intérieur de la chambre de traitement (5) du revêtement de protection (1).
